Europäisches Patentamt

European Patent Office

Office européen des brevets

(19)

(11) Publication number: **0 058 689**

**B1**

(12) **EUROPEAN PATENT SPECIFICATION**

(45) Date of publication of patent specification: **23.04.86**

(51) Int. Cl.⁴: **C 12 N 11/02,** C 12 N 5/00, C 12 N 5/02, C 09 H 7/00

(21) Application number: **81902357.3**

(22) Date of filing: **14.08.81**

(88) International application number:
**PCT/US81/01098**

(87) International publication number:
**WO 82/00660 04.03.82 Gazette 82/07**

(54) **Cell culture medium, improved process of growing animal cells, method of producing microcarriers and microcarriers.**

(30) Priority: **20.08.80 SE 8005838**

(43) Date of publication of application:
**01.09.82 Bulletin 82/35**

(45) Publication of the grant of the patent:
**23.04.86 Bulletin 86/17**

(84) Designated Contracting States:
**FR**

(56) References cited:
**EP-A-0 030 885**
**FR-A-2 464 994**
**NL-A-7 906 064**
**US-A-3 639 558**
**US-A-3 717 551**
**US-A-3 838 007**
**US-A-3 972 776**
**US-A-3 977 941**
**US-A-4 024 020**
**US-A-4 036 693**
**US-A-4 163 691**
**US-A-4 167 447**
**US-A-4 169 761**

(73) Proprietor: **Corning Glass Works**
**Houghton Park**
**Corning New York 14831 (US)**

(72) Inventor: **MOSBACH, Klaus H.**
**Lackalaenga 31-38**
**S-24402 Furulund (SE)**
Inventor: **NILSSON, Kjell G.C.**
**Fagelhundsvagen 30**
**S-22253 Lund (SE)**

(74) Representative: **Reinhard, Skuhra, Weise**
**Leopoldstrasse 51**
**D-8000 München 40 (DE)**

(56) References cited:
**US-A-4 189 534**
**US-A-4 257 884**
**US-A-4 266 029**
**US-A-4 266 032**

(50) References cited:

**Biomedical Applications of Immobilized Enzymes and Protein, vol. 1, plenum press, Issued 1977, Chang, T.M.S. Encapsulation of Enzymes, cell contents, Cells, Vaccines, Antigans, Antiserum, Cofactors, Hormones and proteins, pages 69 and 80-87**

**Chemical Abstracts, Vol. 91, Abstract no. 87596z, Issued 10 September 1979, Brodelius et al, Immobilized plant cells For The production and Transformation of Natural products**

**Chemical Abstracts, vol. 81, Abstract No. 23770n, Issued 05 August 1974, Leighton, J., Collagen-coated cellulose sponge.**

**Chemical Abstracts, vol. 77, Abstract No. 123496p, Issued 06 November 1972, cleator, et al, probable role of Collagen when Used As A growth Surface For Cell culture.**

**Chemical Abstracts, Vol. 88, Abstract No. 132276w, Issued 08 May 1978, Dunn, et al, Contact Guidance On Oriented Collagen Gels.**

**Enzyme Microb, Technol., Vol. 2, Issued January 1980, Halling, et al, Magnetic Supports For Immobilized Enzymes And Bioaffinity Adsorbents. Pages 2-11.**

**Biotechnology and Bioengineering, Vol. IXI, Issued 1977, Kierstan et al, The Immobilization of Microbial Cells, subcellular Organelles and Enzymes In Calcium Alginate Gels, pages 387-397.**

**JOURNAL OF PHARMACEUTICAL SCIENCES, vol. 70, no. 4, April 1981 F. LIM et al.: "Microencapsulation of living cells and tissues", pages 351-354**

## Description

### Technical field

The present invention relates to a cell culture medium, to an improved process of growing anchorage dependent animal cells, to a method of producing microcarriers and to microcarriers.

The surface-area limitations associated with culturing anchorage-dependent animal cells in, for example, roller bottles to a large degree have been overcome through the use of microcarriers. Where the cells *per se* comprise the desired product, however, problems still remain relative to the efficient harvesting of such cells.

### Background art

In the past, there has been considerable interest in the immobilization of cells, particularly those of microbial origin (K. Mosbach, Ed., "Methods of Enzymology," Vol. 44, Academic Press, New York, 1976; and I. Chibata and T. Tosa in L. B. Wingard et al., "Applied Biochemistry and Bioengineering," Vol. 1, Academic Press, New York, 1976, pp, 329—357). More recently, such interest has been extended by the reported immobilization of living plant cells in suspension (P. Brodelius et al., *FEBS Letters, 103*, 93—97 (1979)).

The use of microcarriers for culturing mammalian anchorage-dependent cells in suspension has been given increasing attention in recent years. For example, a DEAE-Sephadex microcarrier was used in some early experiments as a support for the cultivation of fibroblast-like cells derived from embryonic rabbit skin (H cells) and diploid human embryonic lung cells (HEL cells) (A. L. van Wezel, *Nature, 216*, 64—65 (1967)).

Van Wezel has continued to study the use of DEAE-Sephadex microcarriers for the homogeneous cultivation of primary cells and diploid cell strains. See, e.g., A. L. van Wezel "Microcarrier Cultures of Animal Cells," in P. F. Kruse, Jr. and M. K. Patterson, Jr., Editors, "Tissue Culture: Methods and Applications," Academic Press, New York, 1973, pp. 372—377; A. L. van Wezel et al., *Develop. biol. Standard. 42*, 65 (1979), presented at the 2nd General Meeting of ESACT, Paris 1978; and A. L. van Wezel et al., *Process Biochemistry, 13*, 6 (1978).

Interestingly, other workers also concentrated on the use of DEAE-Sephadex microcarriers. For example Levine et al. proposed adding to the culture medium containing DEAE-Sephadex microcarrier beads a negatively charged non-nutritive component, e.g., carboxymethylcellulose, to compete with the positively charged sites on the microcarrier. They also screened various support (microcarrier) materials which appeared to include silica gel and several ion-exchange resins, as well as Sephadex *per se*. The DEAE-Sephadex beads were in the 90—105 μ range. D. W. Levine et al., "Optimizing Parameters for Growth of Anchorage-Dependent Mammalian Cells in Microcarrier Culture", in "Cell Culture and its Applications", Academic Press, New York, 1977, pp. 191—216. See also U.S. Patent No. 4,036, 693 to D. W. Levine et al.

Additional work by D. W. Levine et al. indicated that such microcarrier treatments as that described above can be eliminated if the charge capacity of the microcarrier is adjusted or controlled within a certain range, thereby resulting in good growth of a wide variety of anchorage-dependent cells. Such range is from about 0.1 to about 4.5 milliequivalents per gram of dry material. The microcarriers, however, still appear to be positively charged, and the most suitable microcarrier apparently is DEAE-Sephadex. U.S. Patent No. 4,189,534 to D. W. Levine et al., or British Patent Specification No. 1,535,150 to Massachusetts Institute of Technology. See also D. W. Levine, Ph.D. Thesis, "Production of Anchorage-Dependent Mammalian Cells on Microcarriers", Massachusetts Institute of Technology, 1977.

Of course, materials other than DEAE-Sephadex have been employed as microcarriers. See, for example, U.S. Patent No. 3,717,551 to B. Bizzini et al., which discloses the cultivation of cells on porous silica spherules. Moreover, the immobilization of cells for cultivation purposes has been achieved by means other than through the use of microcarriers, such as by entrapping the cells within a polymeric or gel-like matrix. By way of illustration only, see P. Brodelius et al., *FEBS Letters, 103*, 93 (1979), which describes the entrapment of plant cells within alginate beads.

Finally, the use of gelatin, collagen, and related materials in the cultivation of cells is known. For example, U.S. Patent No. 4,169,761 to P. Precausta et al. discloses the cultivation of cells on collagen fibers which are dispersed or suspended in the nutrient medium. The use of collagen in the form of gel particles for the cultivation of cells on microcarrier surfaces also has been reported; the same report mentions similar uses for cellophane, cellulose sponge, and DEAE-Sephadex, among other materials. C-b. C. Horng, Ph.D. Dissertation, "Primary Culture of Mammalian Cells on Microcarrier Surface" Graduate School, State University of New York at Buffalo, 1975, pp. 4, 5, 136, and 138. See also, J. Leighton, "Collagen-Coated Cellulose Sponge," in P. E. Kruse, Jr., Ed. "Tissue Culture: Methods and Applications," Academic Press, New York, 1973, pp. 367—371 [Chem. Abstr., 81, 2377 On (1974)].

According to French Patent No. 2,419,321 DEAE-Sephadex beads can be pretreated with calf fetus serum to reduce the loss of the inoculum and the lack of reproducibility in cell cultivation precedures using DEAE-Sephadex microcarriers.

It perhaps should be noted that microbial cells have been incorporated in glutaraldehyde-cross-linked gelatin to give an immobilized glucose isomerase. U.S. Patent No. 4,191,810 to N. Yoshikazu et al. Also, the fractionation and manipulation of cells have been accomplished through the use of fiber fractionation techniques—the fiber typically is nylon, optionally coated with gelatin or a gelatin derivative which permits cell recovery by the simple expedient of melting the gelatin or gelatin derivative. See, e.g., G. M. Edelman and U. Rutishauser, "Specific Fractionation and Manipulation of Cells with Chemically Derivatized Fibers and Surface," in W. B. Jakoby and M. Wilchek, Editors, "Methods in Enzymology," Vol. 34, Academic Press, New York 1974, pp. 195—209.

With respect to harvesting or removing cells from microcarriers, the use of trypsin is well known. See, e.g., D. W. Levine et al. in "Cell Culture and Its Applications", Acedemic Press, New York, 1977, p. 207; U.S. Patent Nos. 4,036,693 and 3,717,551; and French Patent No. 2,419,321.

US 4 167 447 (published on 11 September 1979) describes microcarriers composed of chitosan crosslinked with glutaraldehyde (column 2, lines 22—26; column 4, lines 60—66; Example 7, column 7).

Cross-linked gelatin carriers are known from US 3 639 588 published on 1st February 1972 (column 3, lines 44—45; column 4, lines 31—73).

US 4 167 447 mentions glutaraldehyde as a cross-linking agent (column 4, lines 63—64) whereas US 3 639 588 suggests a dialdehyde (column 4, line 64).

NL 7 906 064 (published on 18th February 1980) describes gelatin-coated dense particles, the core of which may contain a material with a density greater than 4 gr/cm³. This document makes reference (page 1, line 10) to US 3 928 143 (published on 23rd December 1975) which mentiones iron (density about 8 g/cm³) and iron oxide (column 5, lines 1—18) among the suitable dense materials.

Finally, the publication "Immobilised plant cells for the production and transformation of natural products" (FEBS letters, 103, (1), 1979, pages 93—97) describes a method to entrap plant cells in alginate gels.

Disclosure of invention

It therefore is an object of the present invention to provide a cell culture medium suitable for use in the immobilization and cultivation of anchorage-dependent animal cells and having a reduced cost.

It is also an object of the present invention to provide a cell culture medium, comprising a microcarrier, suitable for use in the immobilization and cultivation of anchorage-dependent animals cells, from which microcarrier such cells are readily removed if required.

A further object of the present invention is to to provide a magnetic microcarrier suitable for use in the immobilization and cultivation of anchorage-dependent animal cells, which microcarrier has a reduced cost and from which microcarrier such cells are readily removed if required.

Still another object of the present invention is to provide a method for the ready removal of anchorage-dependent animal cells from the microcarriers of the present invention.

These and other objects will be apparent to one having ordinary skill in the art from a consideration of the disclosure and claims which follow.

Accordingly, the present invention provides a cell culture medium, comprising a microcarrier, suspended in said culture medium, suitable for immobilizing cells, comprising crosslinked gelatin particles which can be totally degraded by proteolytic enzymes without significant damage occurring to the cells.

Preferably, the gelatin has been crosslinked with glutaraldehyde.

The present invention also provides an improved process of growing anchorage-dependent animals cells on microcarriers, the improvement comprising:

a) forming the microcarrier from gelatin particles that have been crosslinked; and

b) harvesting intact cells by totally degrading the microcarrier with proteolytic enzymes.

The present invention further provides a method of producing a microcarrier for immobilizing cells comprising:

a) reacting the gelatin particles with a cross-linking agent; and

b) autoclaving the crosslinked gelatin particles, whereby the resulting crosslinked gelatin particles can be totally degraded by a proteolytic enzyme without significant damage occurring to the cells.

The present invention further provides the microcarriers of claims 5 and 7—11.

Brief description of drawings

Figure 1 illustrates the growth of two primary cell cultures on the gelatin beads of the present invention.

Figure 2 illustrates the release of microcarrier-bound cells using trypsin, collagenase, and dispase, respectively.

Modes for carrying out the invention

In the course of our studies to obtain a suitable gel matrix for entrapment of animal cells, we found, in analogy to T. Elsdale and J. Bard, *J. Cell. Biol.*, *54*, 626—637 (1972), that collagen substrate adhered to cells. On further investigation, a simple technique leading to the preparation of solid beads, 100—250 µm, of the closely related gelatin (the product obtained on boiling collagen) was developed. All the cells tested attached and proliferated. In this context, magnetic gelatin beads were also prepared and, as expected, no adverse effect on cell growth was observed. Potentially, such supports would permit facile recovery when used in media of viscous or particulate nature. Furthermore, it is conceivable to suspend the beads by applying an outer magnetic field, thereby minimizing the need for vigorous agitation when using carriers of high specific weight.

In our experiments involving detachment of cells, we attempted an alternative approach as there was the possibility with the new microcarriers of the invention used to enzymically dissolve the support directly. Both dispase and collagenase dissolved the beads, leading in the case of dispase to a clear solution. In contrast to trypsin treatment which involves destruction of the cell surface to accomplish cell removal, the approach taken here should leave the entire cells intact and viable which is an advantage, e.g., when the cells are used for immunization or when analysis of surface antigens will be done. Summerizing, as gelatin is inexpensive and represents a more 'natural' surface for cell attachment than those commercially available, it appears that the microcarriers of the invention will turn out to be valuable supports for cell growth as also strongly indicated by the spinner culture experiments reported here. The ease of cell release adds to the merits of such microcarriers. Furthermore, the microcarriers can be degraded substantially completely, thereby preventing the readsorption of the cells.

The present invention is further illustrated by the example which follows. Such example, however, is not to be construed as limiting the present invention in any manner.

Example
A. Materials and method
A.1. Materials

Collagenase (type I, 190 U/mg) was obtained from Sigma. Glutaraldehyde (25%) was from Merck AG. The magnetic particles, $Fe_3O_4$ (5 µm), were a gift from Höganäs, Sweden. Arlacel 83 was obtained from Atlas Chemical Co., dispase (grade II, 0.5 U/mg) was from Boehringer. Gelatin (commercial grade) was from Kebo, Sweden.

A.2. Cell types

The following cells were kindly provided by Professor O. Sjögren: the primary cell cultures S 157N (human skin fibroblasts), S 158A (human kidney carcinoma), DMH W49 (rat colon carcinoma) and DMH W1073 (rat colon carcinoma). All cells were grown in Waymouth media supplemented with 20% fetal calf serum. All media were supplemented with gentamicin (50 mg/l).

A.3. Gelatin microcarriers

A gelatin solution (10 ml; 20% (w/v)) obtained by heating to 50°C was dispersed under vigorous stirring in 100 ml of a mixture of toluene:-chloroform (73/27, v/v) containing 2% (w/v) of Arlacel 83 at room temperature. After 10 min the mixture was filtered through a 100 µm nylon net whence the collected microcarriers were transferred to acetone. After careful washing with acetone the microcarriers were evaporated to dryness. In order to obtain beads which would resist higher temperatures, cross-linking with glutaraldehyde was carried out. Dry beads (1.5 g) were reswollen in 100 ml water followed by the addition of 20 ml 25% glutaraldehyde. After gentle stirring for 30 min the beads were collected and washed with 0.15 M NaCl on a 100 µm nylon net. The beads were dispersed in 0.15 M NaCl and autoclaved at 120°C for 15 min. In order to get a suitable size distribution of the beads, they were first filtered through a 250 µm nylon net and the beads in the filtrate were then collected on a 100 µm net. They were then transferred to 0.15 M NaCl and sterilized through autoclaving at 120°C for 15 min. Magnetic microcarriers were obtained by the same procedure except that in addition to 9 ml 20% (w/v) gelatin solution, 1 g $Fe_3O_4$ particles were added prior to dispersion in the organic phase.

A.4. Test for cell growth

Before use, all microcarriers were washed with 10 vol. medium. Gelatin microcarriers (0.25 g wet wt) were mixed with $2.5 \times 10^5$ cells (S 157N, S 157A, DMH W49 and DMH W1073, respectively) in 5 ml media in 10 ml siliconized test tubes. They were placed in a $CO_2$-incubator (5%) at 37°C and mixed in an end-over-end shaker. The cultures were checked daily and the medium was replaced if necessary. The gelatin microcarriers containing $Fe_3O_4$ were tested with DMH W49. The cells S 157N and DMH W1073 were allowed to grow on gelatin microcarriers (1 g beads, wet wt) suspended in 50 ml spinners. The starting cell concentrations were 35,000/ml and 22,000/ml, respectively; 60% of the medium volume was replaced each day. For determination of the cell number, duplicate samples of 1 ml suspension were taken. After washing with phosphate-buffered saline (PBS) without $Ca^{2+}$ and $Mg^{2+}$, the beads were incubated with trypsin (0.1% in PBS with 0.02% EDTA and without $Ca^{2+}$ and $Mg^{2+}$) for 15 min at 37°C and the released cells counted in a Bürker chamber.

A.5. An alternative method for harvesting of cells

After 6 days the spinner culture of DMH W1073 was divided into 3 equal fractions after washing as above. They were incubated with 3 ml enzyme solution, trypsin as described above and collagenase (2 mg/ml) in PBS with $Ca^{2+}$ but without $Mg^{2+}$ and dispase (4 mg/ml) in Waymouth medium at room temperature. Samples were taken at intervals and the cell concentration determined.

B. Results
B.1. Cell attachment and growth

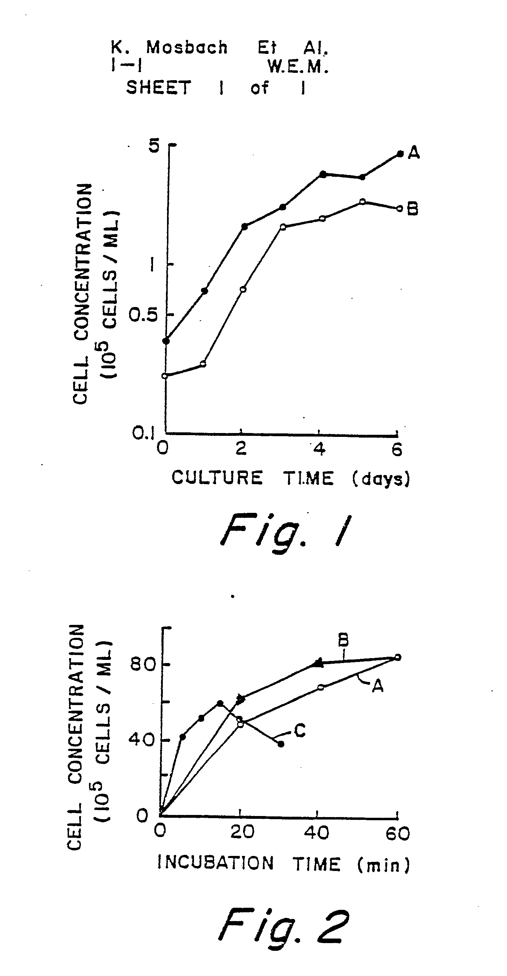

In a preliminary test, various cells were tested for their capacity to attach to different microcarriers (Table 1). All cells would attach to the various beads. Studies on cell growth were subsequently carried out with the most promising microcarrier, gelatin beads with the cells S 157N and DMH W1073. Upon plotting cell concentrations for the two primary cell cultures S 157N and DMH W1073 grown on gelatin beads versus culture time, it is seen that good growth occurred with no indication of cell death. Such plot is shown in Figure 1, which is based on the mean of two cell counts; curve A is for S 127N and curve B is for DMH W1073.

TABLE 1
Attachment of primary cell cultures
to different microcarriers

| | S 157N | S 158A | DMH W49 | DMH W1073 |
|---|---|---|---|---|
| Gelatin | + | + | + | + |
| Gelatin (magnetic) | n.t. | n.t. | + | n.t. |

n.t., not tested

B.2. Harvesting of cells

In Figure 2, the results of three different enzymic procedures leading to cell detachment from gelatin microcarriers are given. As seen, both collagenase (curve A) and dispase (curve B) gave rise to a far higher total cell concentration than normally applied trypsin treatment.

**Claims**

1. A cell culture medium, comprising a microcarrier, suspended in said culture medium, suitable for immobilizing cells, comprising crosslinked gelatin particles which can be totally degraded by proteolytic enzymes without significant damage occurring to the cells.

2. A cell culture medium, according to claim 1, wherein the gelatin has been crosslinked with glutaraldehyde.

3. An improved process of growing anchorage-dependent animals cells on microcarriers, the improvement comprising:

a) forming the microcarrier from gelatin particles that have been crosslinked; and

b) harvesting intact cells by totally degrading the microcarrier with proteolytic enzymes.

4. A method of producing a microcarrier for immobilizing cells comprising:

a) reacting the gelatin particles with a crosslinking agent; and

b) autoclaving the crosslinked gelatin particles, whereby the resulting crosslinked gelatin particles can be totally degraded by a proteolytic enzyme without significant damage occurring to the cells.

5. A microcarrier produced by the method of claim 4.

6. The method of claim 4 wherein the autoclaving is performed at about 120°C for about 15 minutes.

7. A microcarrier produced by the method of claim 6.

8. The process recited in claim 3 wherein the crosslinked gelatin has been autoclaved.

9. A microcarrier of claim 1, 2, 5 or 7, characterized in that said microcarrier contains particles of a magnetic material.

10. A microcarrier of claim 9, characterized in that said magnetic material consists essentially of Fe$_3$O$_4$.

11. The microcarrier of claim 1, 2, 5, 7, 9 or 10 characterized in that the microcarrier is degradable by dispase or collagenase.

**Patentansprüche**

1. Zellkulturmedium, umfassend einen Mikroträger, der in dem Zellkulturmedium suspendiert ist, geeignet zum Immobilisieren von Zellen und vernetzte Gelatine-Partikel umfassend, welche durch proteolytische Enzyme vollständig abbaubar sind, ohne daß eine wesentliche Beschädigung der Zellen auftritt.

2. Zellkulturmedium nach Anspruch 1, in welchem die Gelatine durch Glutaraldehyde vernetzt ist.

3. Verbessertes Verfahren zum Aufbringen von adhäsionsabhängigen Tierzellen auf Mikroträger, wobei die Verbesserung in folgenden Verfahrensschritten besteht:

a) Formen eines Mikroträgers aus Gelatine-Partikeln, die vernetzt wurden; und

b) Ernten intakter Zellen durch vollständigen Abbau des Mikroträgers mit proteolytischen Enzymen.

4. Verfahren zur Herstellung eines Mikroträgers für das Immobilisieren von Zellen, umfassend die folgenden Verfahrensschritte:

a) Umsetzen der Gelatine-Partikel mit einem Vernetzungsmittel; und

b) Autoklavieren der vernetzten Gelatine-Partikel, wobei die resultierenden vernetzten Gelatine-Partikel vollständig durch proteolytische Enzyme abgebaut werden können, ohne daß eine signifikante Beschädigung der Zellen auftritt.

5. Mikroträger, hergestellt gemäß dem Verfahren nach Anspruch 4.

6. Verfahren nach Anspruch 4, wobei das Autoklavieren bei Temperaturen von ca. 120°C und über eine Zeitdauer von ca. 15 Minuten durchgeführt wird.

7. Mikroträger, hergestellt gemäß dem Verfahren nach Anspruch 6.

6. Verfahren gemäß Anspruch 3, bei welchem die vernetzte Gelatine autoklaviert wurde.

9. Mikroträger nach Anspruch 1, 2, 5 oder 7, dadurch gekennzeichnet, daß der Mikroträger Partikeln aus magnetischem Material enthält.

10. Mikroträger nach Anspruch 9, dadurch gekennzeichnet, daß das magnetische Material im wesentlichen aus Fe$_3$O$_4$ besteht.

11. Mikroträger nach Anspruch 1, 2, 5, 7, 9 oder 10, dadurch gekennzeichnet, daß der Mikroträger durch Dispase oder Collagenase abbaubar ist.

**Revendications**

1. Milieu de culture de cellules, comprenant un microporteur en suspension dans ledit milieu de culture, se prêtant à l'immobilisation de cellules, comprenant des particules de gélatine réticulée pouvant être totalement dégradées par des enzymes protéolytiques sans endommagement significatif des cellules.

2. Milieu de culture de cellules selon la revendication 1, dans lequel la gélatine a été réticulée par du glutaraldéhyde.

3. Procédé améloiré pour la croissance de cellules animales dépendant de leur mode de fixation, sur des microporteurs, le perfectionnement comprenant:

a) la formation de microporteurs à partir de particules de gélatine ayant été réticulées; et

b) le recueil de cellules intactes par dégradation totale du microporteur par des enzymes portéolytiques.

4. Procédé de production d'un microporteur pour l'immobilisation de cellules comprenant:

a) la réaction des particules de gélatine avec un agent de réticulation; et

b) le passage à l'autoclave des particules de gélatine réticulées, les particules de gélatine réticulées obtenues pouvant être totalement dégradées par une enzyme protéolytique sans endommagement significatif des cellules.

5. Microporteur produit par le procédé de la revendication 4.

6. Procédé selon la revendication 4, dans lequel le passage à l'autoclave est effectué à environ 120°C pendant environ 15 minutes.

7. Microporteur produit par le procédé selon la revendication 6.

8. Procédé selon la revendication 3, dans lequel la gélatine réticulée a été passée à l'autoclave.

9. Microporteur selon la revendication 1, 2, 5 ou 7, caractérisé par le fait que ledit microporteur contient des particules d'un matériau magnétique.

10. Microporteur selon la revendication 9, caractérisé par le fait que ledit matériau magnétique est essentiellement constitué de $Fe_3O_4$.

11. Microporteur selon la revendication 1, 2, 5, 7, 9 ou 10, caractérisé par le fait que le microporteur est dégradable par la dispase ou la collagénase.

Fig. 1

Fig. 2